# EUROPEAN PATENT APPLICATION

(11) **EP 0 960 623 A2**
(43) Date of publication of application: **01.12.1999**
(21) Application number: 99600002.2
(22) Date of filing: 13.01.1999
(51) Int. Cl.: A61K 51/00

(54) **Kit for the single step preparation of pentavalent technetium 99mTc(V)-DMSA**

(30) Priority: 13.01.1998 GR 98000018
(71) Applicant: N.C.S.R. "DEMOKRITOS"-Institut of Radioisotopes & Radiodiagnostic Products, 153 10 Aghia Paraskeyi Attikis (GR)
(72) Inventor: Efstratios, Chiotellis, Dr., 11635 Athens (GR)

(57) **Abstract**

Technetium 99m (V)-dimercaptosuccinic acid (DMSA) is a diagnostic imaging agent with clinical application in the diagnosis of myeloid thyroid carcinoma, of bone and soft tissue tumors and breast tumors. Furthermore, significant retention in bone metastases has been reported.

The invention concerns a standardized formulation of a freeze-dried kit for the single-step preparation of the radiopharmaceutical pentavalent technetium-^{99m}Tc(V)-dimercaptosuccinic acid only by the reconstitution of the freeze-dried kit content by adding an appropriate volume of injectable solution of sodium Pertechnetate-^{99m}Tc.

Consequently, the kit formulation is developed to contain all necessary constituents in a stable in vitro composition which enables the kit storage for three months. The vial content - lyophilized dry powder - is sterile and pyrogen free.

The method of the kit preparation comprises the mixture of two DMSA solutions (A and B) of different compositions and acidity. The final solution is prepared by adding solution B to solution A; Lyophilization is taking place at zero degrees Celsius for 24 hours, whereas vials are sealed inside the lyophilization chamber under dried and sterile nitrogen 99.999% pure. The whole procedure must be run under aseptic conditions. The cold kit should be stored in the freezer.

The kit labeling with technetium-99m is achieved with the reconstitution or total dissolution of the kit content by adding 2.5 ml of sodium pertechnetate-Tc99m injectable solution of total radioactivity between 0.74 - 2.96 GBq (20-80 mCi).

The method of labeling is therefore performed simultaneously with the kit reconstitution. Therefore the preparation of the pentavalent technetium 99m(V)-dimercaptosuccinic acid is a very simple procedure, easy-to-use, efficient and pharmaceutically safe.

The prepared solution of pentavalent technetium 99m(V)- dimercaptosuccinic acid may be used for 2.5 hours after labeling when kept in the refrigerator (preservation conditions) conforming to the rules of protection against radiation.

## Description

### Technical Field

The objective of the invention comprises of methods, compounds, formulations and cold kits of the radiopharmaceutical pentavalent ^{**99m**}Tc(V) - dimercaptosuccinic acid (DMSA), which has clinical application in the imaging of myeloid thyroid carcinoma, of bone and soft tissue tumors and of breast tumors in nuclear medicine diagnosis.

An objective of the invention is the formulation of a cold kit for the single-step preparation of a radiopharmaceutical and the method of its preparation.

Another objective of the invention is the cold kit preparation which is consisted of a vial containing lyophilized product suitable for injection after reconstitution, ready to be labeled with technetium-99m in a single-step, only with addition of injectable solution of sodium Pertechnetate-99mTc.

Another objective of the invention is the method of kit labeling (labeling with radioactive technetium-99m). The nuclear physician will have only to add a defined volume of sodium Pertechnetate-^{99m}Tc injectable solution, so that in a single step only, the complex ^{99m}Tc(V)-DMSA is formed in a form that is pharmaceutically accepted for intravenous administration.

### Disclosure of the invention

### The prior state-of-the-art

The compound ^{99m}Tc(V)-DMSA is a diagnostic imaging agent (Yokohama, 1985) with clinical application in the diagnosis of the myeloid thyroid carcinoma (Ohta, 1984), of bone and soft tissue tumors (Kobayashi, 1994) and breast tumors (Nakamoto, 1997). Furthermore, significant retention during bone metastases has been reported (Ohta, 1988).

Technetium-99m can form various complexes with the dimercaptosuccinic acid (DMSA). The pentavalent technetium complex ^{99m}Tc(V)-DMSA is chemically different from the well known trivalent ^{99m}Tc(III)-DMSA which is clinically used for the diagnostic imaging of kidneys, although the ligand (DMSA) is the same.

The structure and chemical identity of the pentavalent compound ^{99m}Tc(V)-DMSA is known (Blower, Singh & Clarke, 1991) and consists of a mixture of three complex isomers (see Figure 1).

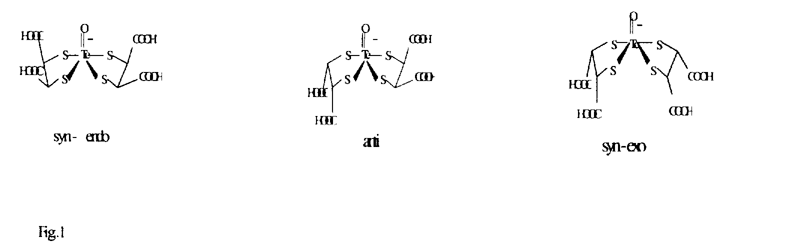

It is considered so far that ^{99m}Tc(V)-DMSA is in vivo dissociated due to its hydrolysis into the cancer cells, so that the pentavalent anion TcO₄³⁻ is formed, which behaves similarly to phosphoric ions PO₄³⁻ (Yokohama, 1981). However this mechanism has not been experimentally proven.

It is also supported that the concentration mechanism of 99mTc(V)-DMSA in various pathological cases depends on the pH values, according to studies implemented on rats with Ehrlich tumors, which show the complex to be concentrated in the cancer cells after acidification with glucose (Horiuchi, 1995).
Based on the above assumption, different methods have been followed for the complex preparation, involving either the in situ preparation or a two-step method (Ohta, 1984, Westera, 1985). Yokohama (1980-1981), Endo (1983) and Hata (1983) have studied the pharmaceutical and clinical behavior of a ^{99m}Tc(V)-DMSA complex which was prepared in a slightly alkaline pH in the presence of low concentrations of stannous chloride (SnCl₂).

The in situ preparation of the complex compound ^{99m}Tc(V)-DMSA was applied clinically for several decades and was based on the preparation of an injectable solution of the complex by in situ mixing the DMSA solution with a suitable reducing agent in an alkaline pH and then by adding the trivalent complex ^{99m}Tc(III)-DMSA designated for the kidneys imaging.

According to the more recent two-step method the ^{99m}Tc(V)-DMSA preparation is produced by addition of sodium bicarbonate solution (NaHCO₃ ) in a DMSA renal kit used for the preparation of the trivalent DMSA complex. After the kit is reconstituted by alkaline solution, sodium pertechnetate-^{99m}Tc is added. The whole procedure must be kept aseptic, whereas particular attention is necessary to maintain inert gas atmosphere into the vial taking into account that the complex ^{99m}Tc(V)-DMSA is easily oxidized in air.

Both methods present significant disadvantages regarding the assurance of pharmaceutical quality and radiochemical purity. The purity of the pentavalent complex must be tested by thin layer chromatography prior to administration (≥95%) whereas free pertechnetate-^{99m}Tc or other technetium derivatives must not be detected. Images with a conventional γ- camera are taken 120 minutes after i.v. administration of 10-20 mCi ^{99m}Tc(V)-DMSA.

### Advantages of the invention referring to the prior state-of-the-art.

The invention concerns the formulation of a cold kit which enables the preparation of the radiopharmaceutical compound ^{99m}Tc(V)-DMSA only in one step. The preparation of the pentavalent technetium ^{99m}Tc-DMSA is implemented by the simple reconstitution of the kit content with the addition of injectable sodium Pertechnetate-^{99m}Tc solution.

Labeling with technetium is performed simultaneously with the reconstitution of the vial content by adding injectable sodium Pertechnetate-^{99m}Tc solution only.

The method is particularly simple, easy-to-use and effective and ensures high radiochemical yield not less than 95% (^{99m}Tc(V)-DMSA) and therefore reduces the possibility of human error. Also, the method of labeling assures conformity with sterility specifications during reconstitution and intravenous administration to the patient, as well as protection of the medical personnel against radiation.

The kit formulation comprising of all the necessary constituents is stable in vitro for three months and enables the kit to be stored for a specific period of time, thus facilitating the clinical practice. The method of the kit preparation assures the pharmaceutical quality, safety and efficiency of the radiopharmaceutical formed after labeling, which has to be sterile and pyrogen free.

### Detailed description of the invention

The preparation of a cold kit is described (in the form of lyophilized powder for reconstitution) which enables the preparation of ^{99m}Tc(V)-DMSA in one single step, namely the reconstitution with sodium pertechnetate-^{99m}Tc solution (single composition instant freeze dried kit).

As a kit, a standardized pharmaceutical formulation is considered, consisted of one or more vials with suitable composition in order to be administered intravenously. The content of the kit has the pharmaceutical form of lyophilized powder and is sterile and pyrogen free.

The kit is a formulation for the single step preparation of the complex ^{99m}Tc(V)-DMSA and contains all the necessary constituents in a stable in vitro composition.

The method of the kit preparation is very simple, easy-to-use, effective and pharmaceutically accepted enabling the storage of the cold kit for a suitable period of time (3 months), thus facilitating significantly the clinical routine.

DMSA forms a chelate compound with technetium-99mTc in the presence of a suitable reducing agent (stannous chloride, SnCl₂), in order to reduce the septavalent Technetium to pentavalent in one single step and in pH 7-8.

The labeling consists of the reconstitution of the kit content with the addition of the needed volume (2.5 ml) of sodium pertechnetate-^{99m}Tc solution (oxidation state +7) in normal saline solution. The method of labeling results to radiochemical purity not less than 95% (^{99m}Tc(V)-DMSA).
The metastable technetium [^{99m}Tc] decays with the emission of gamma radiation to the quasistable form [⁹⁹Tc] with an energy of 140.5 keV and a half life of 6,007 hours. The atomic number of technetium is 43 and its mass number is 99m.

In order to understand fully the pharmacology, the in vivo behavior and the retention mechanism of ^{99m}Tc(V)-DMSA, a biodistribution study on rats was performed, with the method of autoradiography of selected tissues (kidneys and liver). Furthermore, pre-clinical studies were conducted on volunteers. In parallel, a study was conducted of the in vitro stability and the radiochemical impurities determination during the process of the pentavalent complex single step formation.

Each vial contains: 1.36 mg DMSA, 0.105 mg anhydrous stannous chloride 12.5 mg inositol, 0.175 mg ascorbic acid, 10.00 mg dextrose and 16.52 sodium bicarbonate.

Stannous chloride is used as a reducing agent of technetium to the oxidation state Technetium (+5) in acidic pH. Ascorbic acid acts as antioxidant in acidic environment, thus supporting the activity of stannous chloride as a reductant. The role of ascorbic acid as antioxidant is significant. Formulations developed in the absence of ascorbic acid gave low labeling efficiency of ^{99m}Tc. Inositol combined with dextrose offers the best results during the lyophilization process by forming a compact and completely dry product (pellet).

| **Composition of active ingredients per vial** | | | | |
|---|---|---|---|---|
| **No.** | **Name** | **Activity** | **Quantity/vial** | **Units** |
| 1. | Dimercaptosuccinic acid (DMSA) | Active ingredient | 1,36 | mg |
| 2. | Anhydrous stannous chloride (SnCl2) | Reducing agent | 0,105 | mg |

| **Composition per vial - Other constituents and excipients** | | | | |
|---|---|---|---|---|
| **No.** | **Name** | **Activity** | **Quantity/vial** | **Units** |
| 1. | Inositol | Stabilizer | 12,5 | mg |
| 2. | Ascorbic acid | Antioxidant | 0,175 | mg |
| 3. | Dextrose | Preservative | 10,0 | mg |
| 4. | Sodium bicarbonate | Reducing agent | 16,52 | mg |
| 5. | Water for injection (prior to lyophilization) | Solvent | 1 | ml |
| TOTAL WEIGHT | | | 40,66 | mg |

### Method of kit preparation

The method of the kit preparation comprises of mixing the two DMSA solutions having different pHs (solutions A and B) under nitrogen. The final solution is produced by addition of solution B into solution A.

Solution A (the acidic DMSA solution) contains 50 mg DMSA, 35 mg ascorbic acid and 2,5 mg inositol in 100 ml of water to which 1,5 ml of acidic stannous chloride solution is added - 70 mg in 5 ml concentrated hydrochloric acid - pH is adjusted to 2,2 with dilute sodium hydroxide solution.

Solution B (the alkaline DMSA solution) contains 2 g dextrose and 3,304 g sodium bicarbonate in 100 ml of water, to which 222 mg DMSA are added. The final pH is about 8,0.

In continuance, solutions A and B are mixed to form the final solution, which is distributed in volumes of 1 ml into pre-sterilized vials which are lyophilized at zero degrees Celsius for 24 hours.

The lyophilized kit is produced under sterile conditions and stored in the freezer (minus 10 degrees Celsius).

In more details:

### Preparation of solution A:

A 70 mg solution of stannous chloride is prepared in 5 ml of concentrated hydrochloric acid. The solution is stirred until the content is completely dissolved and then is filtered into a pre-sterilized baker through a Millipore pre-tested filter (0,22 µm). Fifty grams of DMSA are brought into 60 ml of injectable water in a baker following by 2,5 grams of inositol and the mixture is heated slightly under stirring and nitrogen bubbling until is completely dissolved. Then, 1,5 ml of the stannous chloride solution in concentrated hydrochloric acid and 35 mg of ascorbic acid are added and then stirred until completely dissolved. The whole preparation is performed under nitrogen bubbling. In continuance pH is adjusted to 2,2 with a 5N solution of sodium hydroxide. The solution is diluted up to a volume of 100 ml and is stirred for 30 minutes under nitrogen.

### Preparation of solution B:

DMSA, 222 mg and 2 g of dextrose are dissolved into 80 ml of injectable water oxygen free. The solution is stirred under nitrogen. Then 3,304 g sodium bicarbonate are added and the solution is stirred until completely dissolved. The solution is dissolved with water in a volumetric flask up to a volume of 100 ml and then nitrogen is bubbled.

### Preparation of the final solution:

The final solution is prepared by adding solution B into solution A. Nitrogen is bubbled for 15 minutes and then the solution is filtered through a system of tested Millipore filters. One ml of the solution is distributed into sterilized 10 ml glass vials pre-cooled under liquid nitrogen. Then follows lyophilization at zero degrees Celsius for 24 hours. When the lyophilization is completed, the vials are sealed under 99,999% pure and sterile nitrogen atmosphere inside the lyophilization chamber. In order to ensure complete dryness, the nitrogen must firstly pass through a trap of concentrated sulfuric acid and secondly through another trap of silica gel and anhydrous calcium chloride. Before dried nitrogen is introduced into the lyophilization chamber, it must pass through a 0,22 µm Millipore filter. The nitrogen introduction into the chamber is stopped when a small negative pressure is developed.

Then, the vials are sealed into the lyophilization chamber and therefore contain nitrogen under slight vacuum. In this manner the necessary inert gas atmosphere is retained into the vial and no over-pressure is occurred which might lead to possible contamination with radioactivity when reconstituting the kit.
During the manufacturing process, critical quality parameters are: the quantity of the stannous chloride solution added, the adjustment of pH to 7-8, the monitoring of temperature and time during lyophilization. The kit is stored in the freezer (minus 10 degrees Celsius).

### Method of labeling with radioactive ^{99m}Tc

The method of labeling comprises of the reconstitution of vial content in a single step, only by adding sterile and pyrogen free sodium pertechnetate-^{99m}Tc, as follows:
1. The vial is placed in an upright position into a suitable lead container (for protection against radiation)
2. By using a shielded and sterilized 10 ml needle, a volume of injectable solution of sodium pertechnetate-^{99m}Tc is added with a total radioactivity of 0,74 - 2,96 GBq (20-80 mCi). The volume of the added solution must not exceed 2 to 3 ml.
3. The vial is shaken for one minute until the content is completely dissolved.
4. The vial is left at room temperature for 15 minutes before used until the reaction of the labeling is completed.
5. The radiopharmaceutical is ready for intravenous administration and remains stable for 15 minutes up to 2,5 hours after labeling, stored in the refrigerator (preservation).
6. The yield of labeling and the radiochemical purity of the solution prior to administration must be not less than 95% ^{99m}Tc(V)-DMSA (chromatographic test method, TLC).

The final product (kit) appears as lyophilized dry powder in a glass vial in a inert gas atmosphere. The content of the vial must be sterile and pyrogen free. Bacterial Endotoxins per vial must be less than 175 E.U.

The samples for the stability study performed were stored at -10°C (in the freezer), at 4°C (refrigerator) and at ambient temperature (15-25°C). The kit labeling was performed according to the above described procedure and all the stability tests were implemented during three months period after production (shelf life). The results showed that the radiopharmaceutical meets specifications when kept in the freezer (minus 10°C) for three months. Furthermore, the in vitro stability of the labeled kit was studied and the test results have shown that the radiopharmaceutical remains stable and pharmaceutically acceptable for safe administration within 2,5 hours after labeling, when kept in the refrigerator.

Scintigraphic images with technetium ^{99m}(V)-dimercaptosuccinic acid are taken with a conventional γ-camera, 120 minutes after i.v. administration for diagnostic doses 10-20 mCi (for adults of average weight 70 kg).

## Claims

1. A method of preparation of a cold kit formulation for the single step preparation at pH 7-8 of the complex pentavalent technetium ^{99m}Tc(V)-dimercaptosuccinic acid (DMSA) after reconstitution of the kit content by adding sodium Pertechnetate-^{99m}Tc solution, which must be sterile, pyrogen free containing no oxidants (labeling with radioactive technetium-^{99m}Tc).

2. A method of labeling the kit according to claim 1, in which labeling is conducted with radioactive technetium-^{99m}Tc or any other suitable radionuclide with similar chemical behavior, through the reconstitution and complete dissolution of the vial content by adding a suitable volume of injectable solution of sodium Pertechnetate-^{99m}Tc (Na^{99m}TcO₄) in normal saline solution (0,9% sodium chloride w/v).

3. A method of labeling according to claim 2, which is conducted as follows: the vial is placed in a shielded lead container in the upright position. With the use of a sterile and shielded needle of 10 ml, 2,5 ml of sodium Pertechnetate-^{99m}Tc injectable solution with total radioactivity of 0,74-2,96 GBq (20-80 mCi) are added. Two or three (2-3) ml of normal saline solution may be added. The vial is shaken for one minute until the content of the vial is completely dissolved and is kept at room temperature for 15 minutes prior administration for the labeling reaction to be completed.

4. A method for the kit labeling according to claim 3, where sodium pertechnetate-^{99m}Tc solution should not exceed 3 ml in volume and total radioactivity should be 0,74-2,96 GBq (20-80 mCi).

5. A product according to claim 1, which is a standardized cold kit formulation for the single-step preparation of the complex pentavalent technetium ^{99m}Tc(V)-DMSA, only with reconstitution of the kit content by addition of sodium pertechnetate-^{99m}Tc injectable solution (labeling with radioactive technetium-99m).

6. A product (kit) according to claim 5 in the form of standardized pharmaceutical preparation consisted of one or more vials whose content is pharmaceutically accepted for safe intravenous administration, where the vial is sealed under nitrogen and its content is sterile and pyrogen free.

7. A kit according to claim 5 which, based on analytical test studies performed, is in a lyophilized form which enables the storage of the kit presenting statisfactory stability in vitro for three months kept in the freezer.

8. A kit according to claim 5 used for the single-step preparation of the pentavalent ^{99m}Tc(V)-DMSA as described in claims 2 to 4, which is formed after labeling with radioactive technetium-^{99m}Tc at pH 7-8 in the presence of low concentrations of a reducing agent and of a suitable antioxidant.

9. A product (kit) according to claim 5, where the reducing agent according to claim 7 is the stannous chloride (SnCl₂) which in acidic pH environment reduces technetium to the oxidation state (+5).

10. A product (kit) according to claim 5, where the antioxidant according to claim 7 is ascorbic acid in acidic pH environment.

11. A product (kit) according to claim 5, where each vial contains: 1,36 mg DMSA, 0,105 mg anhydrous SnCl₂, 12,5 inositol, 0,175 mg ascorbic acid, 10,00 mg dextrose and 16,52 sodium bicarbonate.

12. A method of preparation of the product (kit) of claim 5, according to which the two solutions of DMSA (A and B) with a different pH, are mixed.

13. A method of preparation of the product (kit) according to claim 12, where the pH of the solution A is acidic (2,2).

14. A method of preparation of the product (kit) according to claim 12, where the pH of the solution B is alkaline (8,0).

15. A method of preparation of the kit according to claim 12, where solution A (acidic DMSA solution) contains in 100 ml of water: 50 g of DMSA, 35 mg of ascorbic acid and 2,5 g of inositol, following the addition of 1,5 ml of prepared acidic solution of SnCl₂ (70 mg in 5 ml of concentrated hydrochloric acid).

16. A method of preparation of the product (kit) according to claim 12, where solution B (the alkaline DMSA solution) contains 2 grams of dextrose and 3,304 grams of sodium bicarbonate in 1000 ml water, to which 222 mg of DMSA are added.

17. A method of preparation of the product (kit) according to claim 12, where the final solution is formed by adding solution B to solution A.

18. A method of preparation of the product (kit) according to claim 12, where each 1 ml of the final solution, whose method of preparation is described in claims 9-17, is distributed in pre-sterilized vials and is lyophilized at zero degrees Celsius for 24 hours.

19. A method of preparation of the product (kit) according to claim 12, where the vials with lyophilized product, as manufactured according to claim 18, are sealed under vacuum and contain dried, inert and sterile gas (nitrogen).

20. A method of preparation of the product (kit) according to claim 12, where the vials with lyophilized product of claim 18 are manufactured under aseptic conditions and stored in the freezer (minus 10 degrees Celsius).

21. A method of preparation of the product (kit) according to claim 12, where more than 95% of the labeled product according to claims 1-2, is in the form of pentavalent technetium ^{99m}Tc(V)-DMSA from 15 minutes up to 2,5 hours after labeling (radiochemical yield of ^{99m}Tc(V)-DMSA), whereas the sum of impurities, ^{99m}Tc(II) and ^{99m}Tc(VII), which may possibly be detected due to the reconstitution with Pertechnetate (^{99m}TcO₄⁻) is less than or equal to 5% and the percentage of the hydrolyzed technetium (^{99m}Tc II) is less than or equal to 2%.

22. A method of preparation of the product (kit) according to claim 12, where the labeled (reconstituted) radiopharmaceutical according to claims 1-2, remains stable and pharmaceutically accepted to be administered within 6 hours after labeling when refrigerated (preservation).

23. The use of the product (kit) according to claim 12, for the single step preparation of the complex pentavalent 99mTc(V)-dimercaptosuccinic acid of claim 1.

24. A product (kit) according to claim 23, which is used as the diagnostic agent for the scintigraphic imaging of the myeloid thyroid carcinoma, bone and soft tissue tumors, breast tumors and other type of tumors.
